(19) Europäisches Patentamt
European Patent Office
Office européen des brevets

(11) EP 0 835 118 B1

(12) **FASCICULE DE BREVET EUROPEEN**

(45) Date de publication et mention de la délivrance du brevet:
**21.05.2003 Bulletin 2003/21**

(21) Numéro de dépôt: **96924019.1**

(22) Date de dépôt: **25.06.1996**

(51) Int Cl.$^7$: **A61K 31/70**, A61K 7/48

(86) Numéro de dépôt international:
**PCT/FR96/00991**

(87) Numéro de publication internationale:
**WO 97/001343 (16.01.1997 Gazette 1997/04)**

(54) **UTILISATION DE SOPHOROLIPIDES POUR LA PREPARATION DE PRODUITS COSMETIQUES, EN PARTICULIER POUR LE TRAITEMENT DE LA PEAU**

VERWENDUNG VON SOPHOROLIPIDEN ZUR HERSTELLUNG KOSMETISCHER PRODUKTE, INSBESONDERE ZUR BEHANDLUNG DER HAUT

UTILIZATION OF SOPHOROLIPIDS FOR THE PREPARATION OF COSMETIC PRODUCTS, IN PARTICULAR FOR THE TREATMENT OF THE SKIN

(84) Etats contractants désignés:
**DE GB IE IT NL SE**

(30) Priorité: **28.06.1995 FR 9508085**

(43) Date de publication de la demande:
**15.04.1998 Bulletin 1998/16**

(73) Titulaire: **SOPHOR S.A.**
**92500 Reuil-Malmaison (FR)**

(72) Inventeur: **MAINGAULT, Martine**
**F-49260 Saint-Macaire-du-Bois (FR)**

(56) Documents cités:

**EP-A- 0 209 783**
**WO-A-95/34282**
**US-A- 4 305 961**

- **PROC. - WORLD CONF. BIOTECHNOL. FATS OILS IND., 1988, 206-9, XP000617067 INOUE, SHIGEO: "Biosurfactants in cosmetic applications" cité dans la demande**
- **JOURNAL OF THE AMERICAN OIL CHEMISTS SOCIETY, vol. 65, no. 9, 1988, pages 1460-1466, XP000566442 ASMER, H-J. ET AL: "Microbial production, structure elucidation and bioconversion of sophorose lipids" cité dans la demande**
- **JOURNAL OF AGRICULTURAL AND FOOD CHEMISTRY, vol. 42, no. 5, 1994, pages 1247-50, XP000617079 KRIVOBOK, S. ET AL: "Production and toxicity assessment of sophorosides from Torulopsis bombicola"**
- **PATENT ABSTRACTS OF JAPAN vol. 7, no. 201 (C-184), 6 Septembre 1983 & JP 58 099498 A (DAIICHI SEIYAKU KK), 13 Juin 1983,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 835 118 B1

Printed by Jouve, 75001 PARIS (FR)

## Description

La présente invention concerne une nouvelle utilisation des sophorolipides appartenant au groupe des glycolipides.

Les sophorolipides encore appelés sophorosides sont généralement obtenus par fermentation d'un substrat au moyen d'une souche bactérienne appropriée. Des procédés de production de ces sophorolipides sont notamment décrits dans le brevet FR-A-2.399.438, la demande internationale PCT/FR91/01027 ou les brevets US-A-3.205.150 et US-A-3.312.684 et dans la publication JALS vol 65, n°9, 1988, 1460-1466, Asmer, H, Jetal; "Microbial production, structure elucidation and bioconversion of sophorose lipids. Ces sophorolipides sont constitués de plusieurs classes. On distingue en particulier les formes lactones acetylées ou non en position 6' et 6" du sophorose et les formes acides acétylées ou non en position 6' du sophorose. La chaîne lipidique varie quant à elle en fonction de la longueur de chaîne des acides la constituant, du nombre et de la position des insaturations et de la position de l'hydroxylation.

La demande de brevet WO 95/34282 décrit et exemplifie les sophorolipides en tant qu'agent de protection des cheveux et de la peau et notamment pour ses propriétés anti-radicalaires, anti-élastasiques et anti-inflammatoires.

Le brevet US 4305 961 décrit une composition cosmétologique comprenant, en tant qu'humidifiant, des groupements hydroxyalkyl éthérifiés sur toutes les fonctions alcool secondaires et sur les groupes acétylés de sophorolipides. Enfin,Inoue, dans Proc World conf. biotechnol fats oil, ind, 1988, 206-9 " Bio-surfactants in cosmetic applications" décrit les sophorolopides en tant qu'humidifiant de la peau.

Les sophorolipides ont déjà été utilisés sous la forme lactone en cosmétique dans le traitement des cheveux pour combattre les pellicules comme le décrit le brevet EP-B209.783 et comme agent bacteriostatique dans les déodorants.

En résumé, ces documents décrivent des applications cosmétologiques des sophorolipides.

Le but de la présente invention est de démontrer que les sophorolipides présentent en outre des activités biologiques élevées s'avérant particulièrement intéressantes pour le traitement de la peau en général et en particulier pour le traitement à des fins esthétiques de la peau.

L'invention concerne à cet effet l'utilisation d'un compose sophorolipidique répondant aux formules générales (1) ou (2) dans lesquelles R1 représente l'hydrogène ou un groupe acétyle, et R2 l'hydrogène ou un reste alkyle comportant 1 à 9 atomes de carbone, lorsque R3 est un reste hydrocarboné saturé à 7 à 16 atomes de carbone, ou bien R2 représente l'hydrogène ou un groupe méthyle lorsque R3 est un reste hydrocarboné insaturé à 13 à 17 atomes de carbones.

L'invention concerne également l'utilisation d'un compose sophorolipidique de la formule générale (1) ayant subi une désacétylation et une estérification du groupe carboxylique.

L'invention concerne plus particulièrement l'utilisation de ce composé sophorolipidique comme substance active dans le traitement cosmétique de la peau.

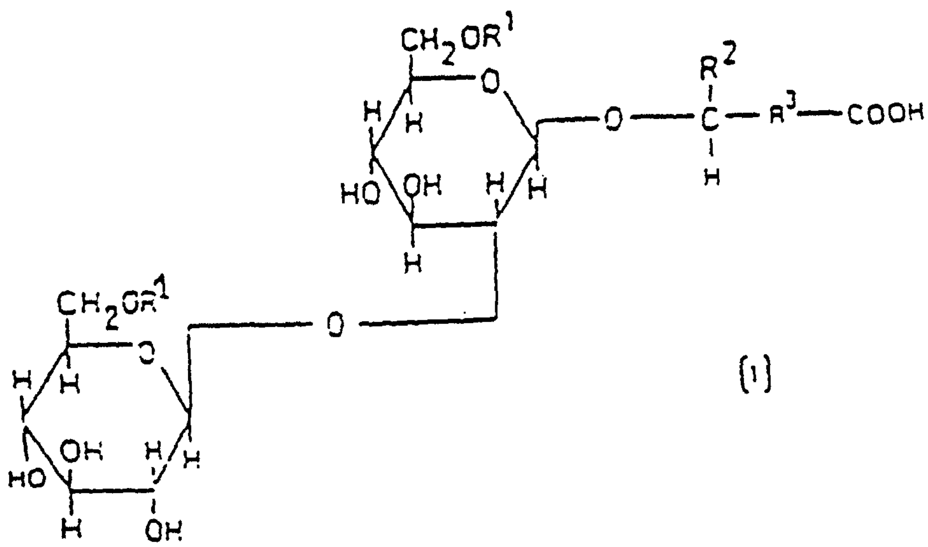

(1)

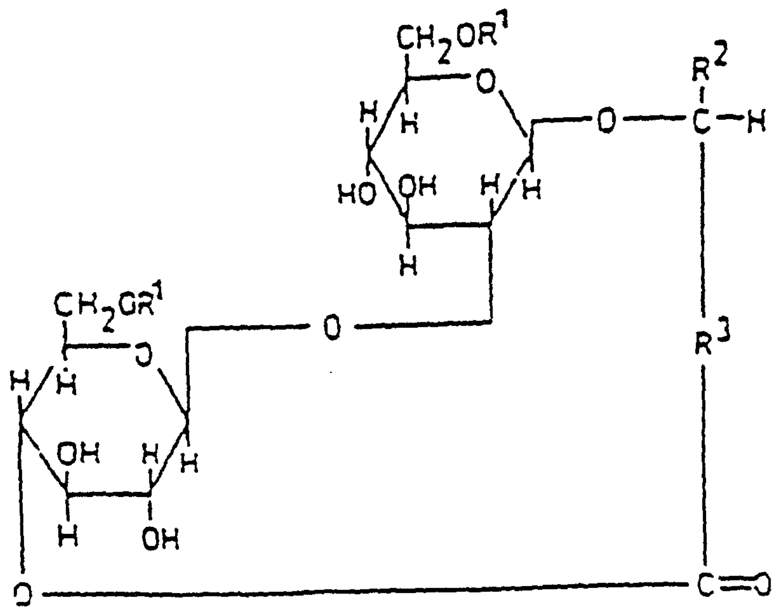

(2)

Selon des formes de réalisation préférées de l'invention, ce composé sophorolipidique peut être utilisé comme agent favorisant la desquamation du fait de son action sur la cohésion des cornéocytes ou bien encore comme agent dépigmentant ou bien encore comme inhibiteur partiel de mélanogénèse en particulier pour le traitement des taches brunes.

L'invention concerne encore des compositions pharmaceutiques caractérisées en ce qu'elles renferment un excipient pharmaceutiquement inerte et en tant que principe actif au moins un composé représenté par les formules (1) ou (2) indiquées ci-dessus, un sel d'au moins un composé de formule (1) indiquée ci-dessus ou au moins un composé de formule (1) désacétylé dont le groupe carboxylique est estérifié.

L'invention concerne enfin l'utilisation d'un composé représenté par les formules (1) ou (2) indiquées ci-dessus, d'un sel d'un composé de formule (1) ou d'un composé de formule (1) désacétylé dont le groupe carboxylique est estérifié, pour l'obtention d'une composition cosmétique destinée à favoriser la desquamation ou la dépigmentation de la peau ou à traiter les tâches brunes à la surface de la peau.

L'invention sera bien comprise à la lecture de la description suivante d'exemples de réalisation en référence aux tableaux annexes dans lesquels :

le tableau 1 représente les résultats d'un test de desquamation et

le tableau 2 représente les résultats d'un test de dosage de la mélanine dans des mélanocytes ayant été placées en contact avec les produits de l'invention.

Comme le montrent les formules développées (1) et (2) ci-dessus, la partie lipidique des sophorolipides peut varier. Ces sophorolipides se présentent d'ailleurs généralement sous forme d'un mélange de différents sophorolipides. Toutefois l'hydroxyacide gras le plus abondant est l'acide 17-hydroxyoctadéc-9-énoique encore appelé acide 17-hydroxyoléïque. En conséquence, tous les essais cités ci-dessous sont réalisés avec un sophorolipide sous forme acide représenté par la formule (a) ou avec un sophorolipide sous forme lactone représenté par la formule (b).

Le sophorolipide (a) aurait pu être également utilisé sous forme de sel ou d'ester. En effet une estérification au moins partielle sur résines H+ du groupement carboxylique d'un sophorolipide désacetylé de formule (1) peut être obtenue par des alcools tels que le méthanol, ce qui conduit à l'obtention d'esters méthyliques de la forme acide totalement désacétylée. En fonction de la nature des composés du mélange, cette estérification peut être précédée d'une désacétylation et

(a)

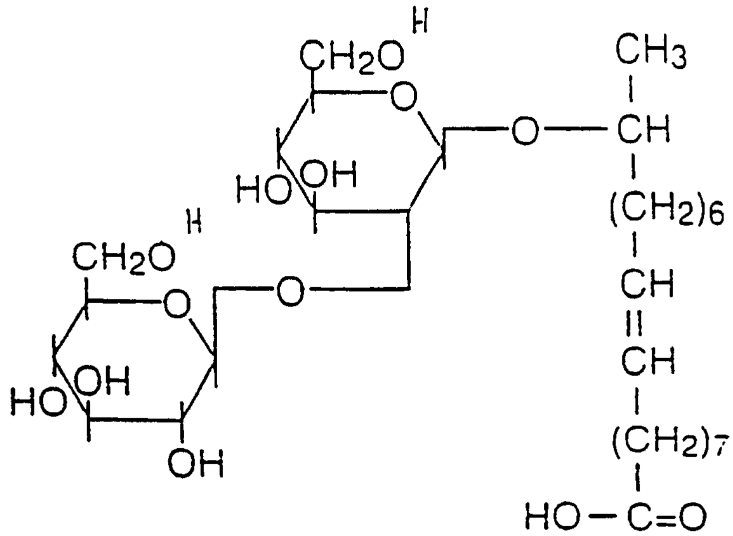

(b)

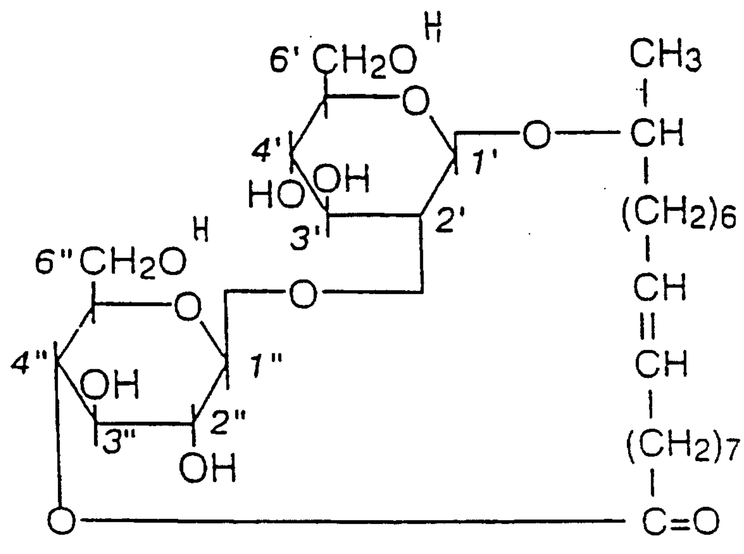

d'une délactonisation sensiblement totales des sophorolipides par saponification suivie d'une neutralisation. Le sophorolipide peut être aussi sous forme d'un sel de métal alcalin ou alcalino-terreux tel que Na, Ca ou Mg.

L'inventeur de la présente invention a constaté que les sophorolipides activent la desquamation physiologique en agissant sur le détachement des cornéocytes. Il est rappelé que l'action desquamante d'un produit consiste à éliminer la partie superficielle de la couche protectrice de l'épiderme (stratum corneum). Cette couche protectrice est constituée de kératinocytes au stade terminal de différenciation appelés cornéocytes. Il était déjà connu que les hydroxy-acides tels que l'acide lactique n'entraînent pas de désagrégation des cornéocytes des couches supérieures du stratum corneum comme le font les agents kératolytiques. En réalité, ils diminuent l'épaisseur de la couche cornée

par une action sur la cohésion cornéocytaire au niveau des couches inférieures nouvellement formées. Ce rôle a également été démontré pour les sophorolipides au moyen des expérimentations qui suivent. Il a été choisi comme modèle cellulaire pour l'étude une culture de kératinocytes placée dans des conditions favorisant la différenciation des cellules. Les actions des sophorolipides forme acide A, des sophorolipides forme lactone B et de l'acide lactique sur le détachement des cornéocytes du modèle cellulaire choisi ont été testées in vitro en parallèle. Les résultats sont fournis dans le tableau 1. Dans chaque expérimentation, il a été déterminé la différence moyenne T60-T0 (cornéocytes/ml) ainsi que le taux de cornéocytes détachés exprimé en pourcentage par rapport au témoin négatif (%/TN). La significativité statistique (test t de Student) des différences entre les échantillons et le témoin négatif est exprimé par p qui demeure inférieur à 0,001 pour tous les essais réalisés. Le coefficient de variation CV est obtenu comme suit :

$$CV = \frac{\text{écart type}}{\text{moyenne}} \times 100$$

La viabilité cellulaire, mesurée par le test au Rouge Neutre est exprimée par rapport au témoin négatif (%). Lorsqu'une dose réponse est réalisée, l'activité du produit peut être définie par la concentration T200. La T200 est la concentration qui entraîne une libération de cornéocytes double de celle observée avec le témoin négatif. Le tableau 4 montre que pour l'acide lactique la T200 correspond sensiblement à une concentration de $10^{-3}$ M alors qu'elle correspond à une concentration de $10^{-4}$ M pour les sophorolipides. Les sophorolipides sont donc dix fois plus actifs que l'acide lactique. Cette action sur la cohésion intercornéocytaire des sophorolipides s'accompagne d'une action sur la flexibilité de la couche cornée et d'une action d'hydratation de l'épiderme. Ces sophorolipides pourront donc être employés en dermatologie dans de multiples indications: ichtyose, acné, xérose, peeling, mais également en cosmétique comme exfoliant ou comme produit anti-rides ou comme produit régénérant. En effet, les applications thérapeutiques impliquent incidemment des applications en cosmétique. Ces applications en cosmétique sont particulièrement surprenantes eu égard à l'application en cosmétique qui avait été décrite dans le brevet EP-B209.783. En effet dans ce document on utilisait le sophorolipides pour combattre les pellicules. Or, on n'utilise généralement pas un produit desquamant pour traiter des pellicules car un effet contraire à l'effet attendu sera obtenu. Cette nouvelle application des sophorolipides en cosmétique déduite de leur utilisation en thérapeutique n'est donc pas évidente au regard de l'application cosmétique déjà connue.

Les formes galéniques des compositions pharmaceutiques ou cosmétiques pour favoriser la desquamation sont diverses. A titre d'exemple, il est fourni ci-après une composition cosmétique favorisant la desquamation de la peau utilisée comme crème régénérante anti-rides :

| | |
|---|---|
| glyceryl stéarate SE | 12 % |
| ceteareth - 12 | 1 % |
| octyldodecanol | 10 % |
| sophorolipide acide | 0,01 à 5 % |
| isopropyl myristate | 6 % |
| huile de germe de blé | 4 % |
| huile de fleur de passion | 3 % |
| glycérine | 5 % |
| eau | qsp 100 |

Parallèlement à cette action de desquamant, il a également été trouvé que les composés sophorolipidiques de l'invention peuvent être utilisés comme inhibiteur partiel de la mélanogénèse et en particulier comme dépigmentant ou pour le traitement des taches brunes en cosmétique. On constate que le vieillissement cutané entraîne un ralentissement des fonctions épidermiques : la mélanine n'arrive plus de façon homogène à la surface de la peau, se concentre à l'intérieur de certaines cellules et des taches brunes apparaissent principalement sur les mains, les avant-bras et le décolleté. Les sophorolipides en agissant sur la mélanogénèse, en particulier en réduisant la production de mélanine, peuvent permettre l'élimination progressive de cette pigmentation. Cette action de dépigmentation est amplifiée par l'effet des sophorolipides sur le détachement des cellules superficielles du fait de leur rôle de desquamant. Pour mettre en évidence, cette action des sophorolipides, il a été procédé comme suit : on a placé en culture des mélanocytes.A J7 et J14, les mélanocytes sont récupérés, lavés (Phosphate Buffer Salin : PBS) et centrifugés (20 000 rpm 20 minutes + 4 °C). Les culots sont repris en PBS/Triton X-100 (1 % v/v) et soniques durant 30 secondes. Le lysat cellulaire est ultracentrifugé (105 000 rpm 40 minutes + 4 °C). Le culot obtenu est alors repris dans 1 ml de PBS. Le taux de protéine est déterminé par méthode calorimétrique (Biorad) puis ajusté à 1,5 mg/ml. Le taux en mélanine est alors déterminé, après hydrolyse par la soude 1N, durant 4 heures à 90 °C, par lecture spectrophotométrique à 420nm

contre une gamme étalon réalisée avec une mélanine de synthèse [Sigma].

Ces résultats sont exprimés en % TN, c'est à dire en pourcentage d'augmentation de synthèse de mélanine calculé comme suit :

100-(teneur en mélanine du produit testé x 100 /teneur en

mélanine TN)

TN correspond au témoin négatif

Le produit testé est soit de l'acide linolénique, acide gras polyinsaturé, soit un sophorolipide acide A. Le tableau 2 montre que contrairement à l'acide linolénique, les sophorolipides forme acide engendrent une réduction de la synthèse de mélanine. Outre les applications au traitement des taches brunes et à la dépigmentation de la peau, plusieurs applications thérapeutiques ou cosmétiques peuvent être envisagées. De ce fait, à nouveau, les formes galéniques sont diverses. Il est toutefois fourni ci-après un exemple d'une crème anti-taches pour les mains utilisée indifféremment en cosmétique ou en thérapeutique :

| | |
|---|---|
| ceteareth 30 | 13 % |
| PEG 7 glyceryl cocoate | 20 % |
| huile de paraffine | 5 % |
| glycérine | 20 % |
| eau | qsp 100 |

Toutes les utilisations précitées des sophorolipides ne sont mises en oeuvre qu'en relation avec une application topique. On constate également que les préparations pharmaceutiques et cosmétiques peuvent avoir des formes très semblables sinon identiques dans certains cas d'utilisation.

Dans le cadre des applications cosmétiques, la concentration en composés sophorolipidiques selon l'invention peut atteindre 0,01 à 35% en poids de matière sèche.

## Tableau 1

### TEST DE DESQUAMATION

| | | Sophorolipide forme acide | | | AC Lactique | Sophorolipide forme lactone | | |
|---|---|---|---|---|---|---|---|---|
| Produits | Témoin | 10-4M | 10-5M | 10-6M | 10-3 M | 10-4M | 10-5M | 10-6M |
| T60-T0 | 1167 | 2278 | 1944 | 1556 | 2500 | 2389 | 1833 | 1528 |
| CV% | 14.29 | 4.22 | 4.95 | 12.37 | 6.67 | 4.03 | 9.09 | 3.15 |
| % /TN | 100 | 195 | 167 | 133 | 214 | 205 | 157 | 131 |
| %viabilité | 100.00 | 98.71 | 99.75 | 99.57 | 99.54 | 99.14 | 100.55 | 98.77 |

**Tableau 2**

| | | | | |
|---|---|---|---|---|
| DOSAGE MELANINE DANS MELANOCYTES APRES 7 ET 14 JOURS | | | | |
| | | | | |
| sophorolipide forme acide → | | | DHA | DHA |
| | %/TN | % TN | % TN | % TN |
| témoin négatif | 100 | 100 | 100 | 100 |
| 10-4M | 56,2 | 56,8 | 109,75 | 113,6 |
| 10-5M | 65,8 | 86,4 | | |
| 10-6M | 93,6 | 95,4 | | |
| | à J7 | à J14 | à J7 | àJ14 |
| | | | | |

% TN = 100 - (Teneur en mélanine du produit testé x 100 / Teneur en mélanine du témoin négatif).

## Revendications

1. Utilisation d'au moins un composé représenté par les formules (1) ou (2),

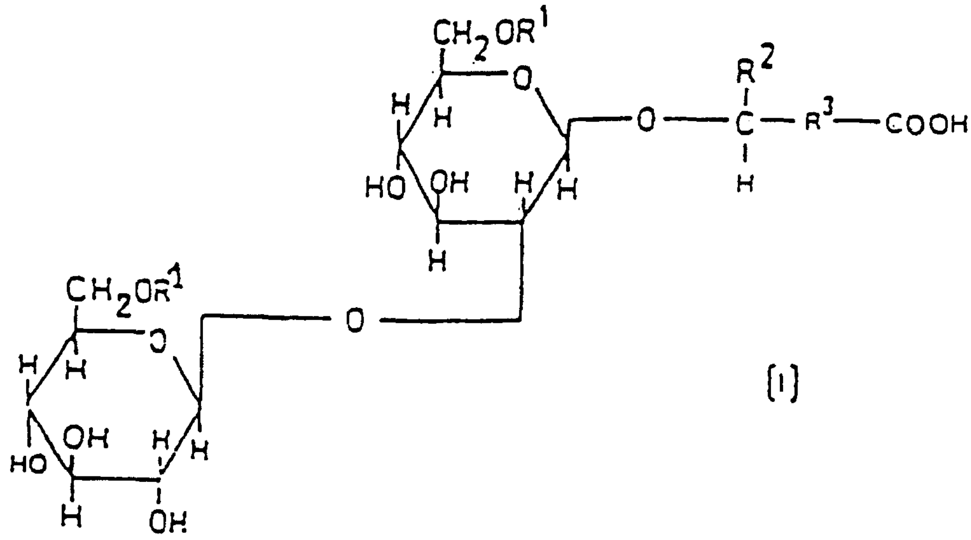

(1)

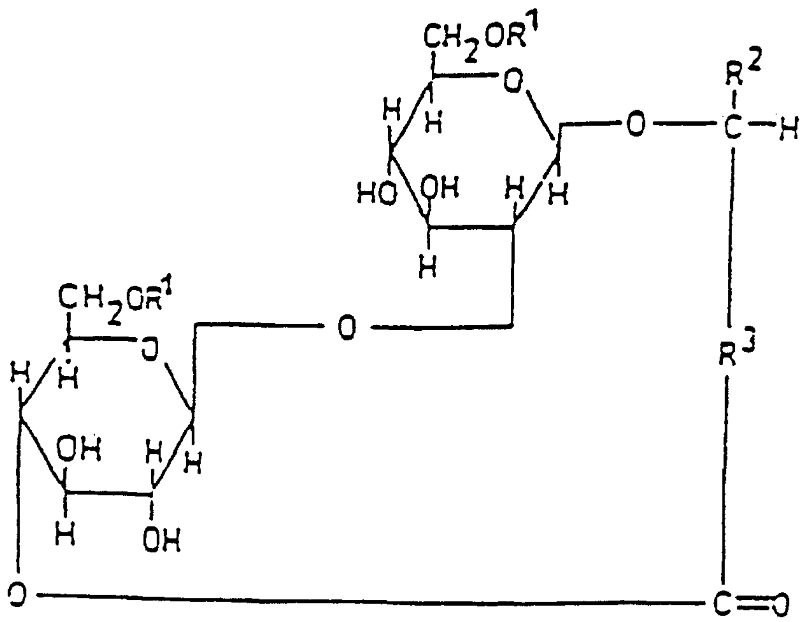

(2)

dans lesquelles R1 représente l'hydrogène ou un groupe acétyle, et R2 l'hydrogène ou un reste alkyle comportant 1 à 9 atomes de carbone, lorsque R3 est un reste hydrocarboné saturé à 7 à 16 atomes de carbone, ou bien R2 représente l'hydrogène ou un groupe méthyle lorsque R3 est un reste hydrocarboné insaturé à 13 a 17 atomes de carbone, d'au moins un sel de composé de formule (1) ou d'au moins un composé de formule (1), désacétylé et dont le groupe carboxylique est estérifié, pour l'obtention d'une composition cosmétique destinée à favoriser la desquamation de la peau.

**2.** Utilisation d'au moins un composé représenté par les formules ( 1 ) ou (2) indiquées dans la revendication 1, d'au moins un sel de composé de formule (1) indiquée dans la revendication 1 ou d'au moins un composé de formule (1) indiquée dans la revendication 1, désacétylé et dont le groupe carboxylique est estérifié, pour l'obtention d'une composition cosmétique destinée à favoriser la dépigmentation de la peau.

**3.** Utilisation selon l'une des revendications 1 et 2 dans laquelle lesdits composés sont destinés au traitement des

taches brunes à la surface de la peau.

**4.** Utilisation selon l'une des revendications 1 et 3 dans laquelle la concentration en composé sophorolipides est comprise entre 0,01 à 35 % en poids des matière sèche.

## Claims

**1.** Use of at least one sophorolipidic compound that corresponds to general formulas (1) or (2)

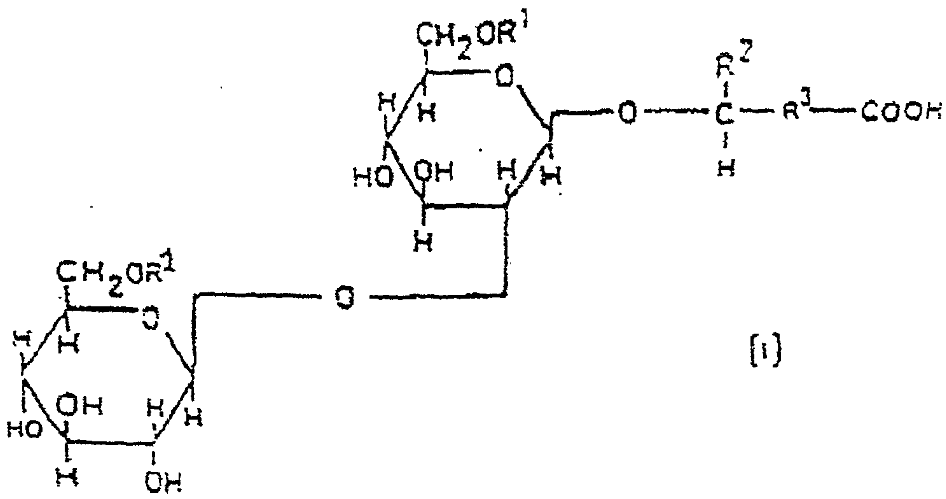

(1)

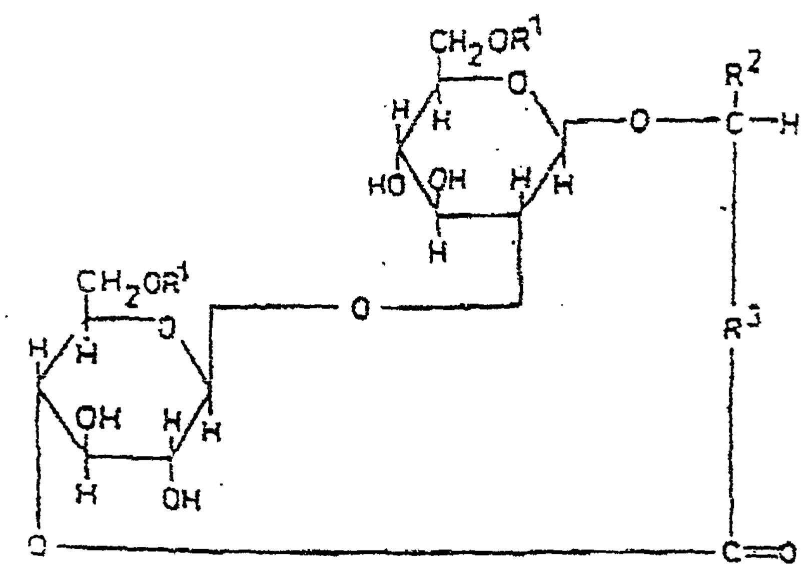

(2)

in which R1 represents hydrogen or an acetyl group and R2 represents hydrogen or an alkyl radical that comprises 1 to 9 carbon atoms, when R3 is a saturated hydrocarbon radical that contains from 7 to 16 carbon atoms, or else R2, represents hydrogen or a methyl group when R3 is an unsaturated hydrocarbon radical that contains from 13 to 17 carbon atoms, of at least one salt of the compound of formula (1) or of at least one compound of formula (1) that is deacetylated and whose carboxylic group is esterified for obtaining a cosmetic composition that is intended to promote desquamation of the skin.

2. Use of at least one compound that is represented by formulas (1) or (2) that are indicated in claim 1, of at least one salt of the compound of formula (1) that is indicated in claim 1, or of at least one compound of formula (1) that is indicated in claim 1 and that is deacetylated and whose carboxylic group is esterified to obtain a cosmetic composition that is intended to promote the depigmentation of the skin.

3. Use according to one of claims 1 and 2, in which said compounds are intended to treat the brown spots at the surface of the skin.

4. Use according to one of claims 1 to 3 in which the concentration of sophorolipid compound is between 0.01 to 35 % by weight of dry material.

**Patentansprüche**

1. Verwendung wenigstens einer Verbindung, dargestellt durch die Formeln (1) oder (2)

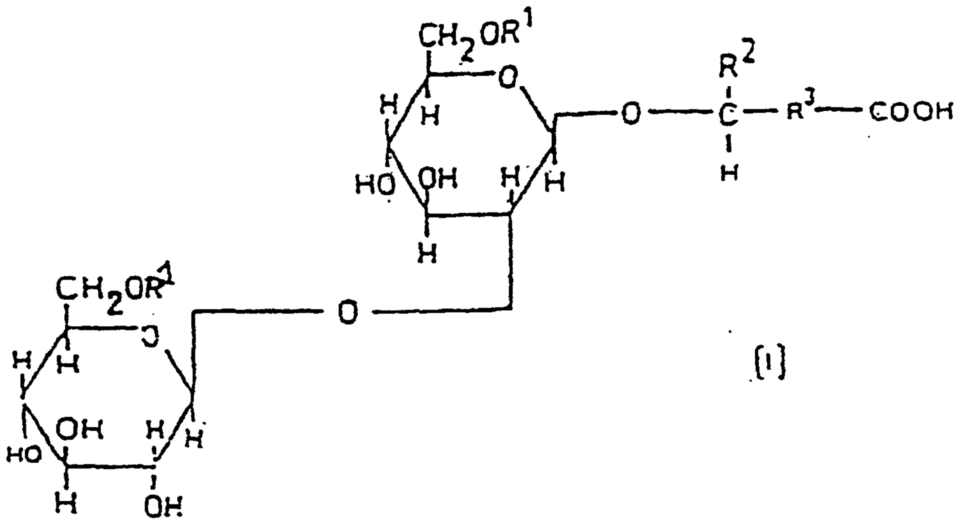

(1)

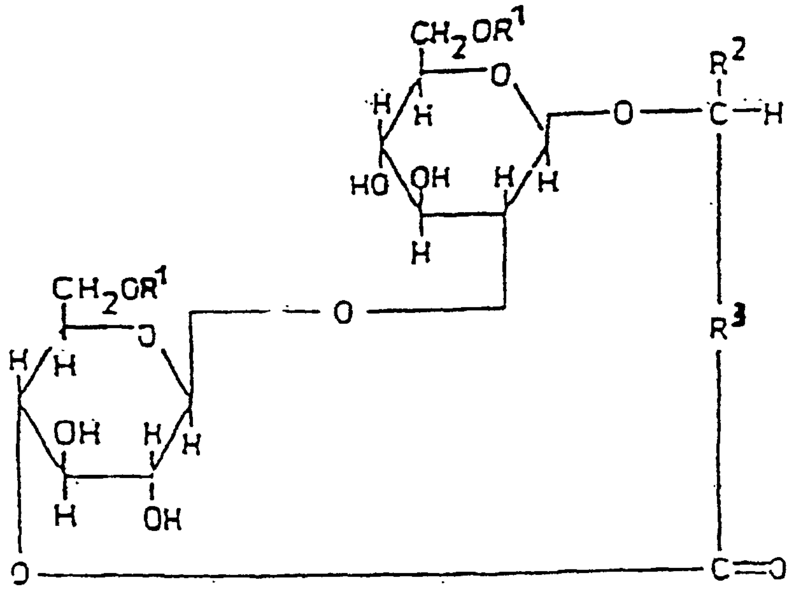

(2)

in denen R1 den Wasserstoff oder eine Acetylgruppe und R2 den Wasserstoff oder einen Alkylrest mit 1 bis 9 Kohlenstoffatomen bedeutet, wenn R3 ein gesättigter Kohlenwasserstoffrest mit 7 bis 16 Kohlenstoffatomen ist, oder auch R2 den Wasserstoff oder eine Methylgruppe bedeutet, wenn R3 ein ungesättigter Kohlenwasserstoffrest mit 13 bis 17 Kohlenstoffatomen ist, wenigstens eines Salzes der Verbindung der Formel (1) oder wenigstens eine Verbindung der Formel (1), die deacetyliert ist und deren Carboxylgruppe esterifiziert ist, um eine kosmetische Verbindung zu erhalten, die dazu bestimmt ist, die Desquamation bzw. Abschuppung der Haut zu begünstigen.

**2.** Verwendung wenigstens einer durch die in Anspruch 1 angegebenen Formeln (1) oder (2) dargestellten Verbindung, wenigstens eines Salzes der Verbindung der Formel (1), angegeben in Anspruch 1, oder wenigstens einer Verbindung der Formel (1), angegeben in Anspruch 1, die deacetyliert ist und deren Carboxylgruppe esterifiziert wird, um eine kosmetische Verbindung zu erhalten, die dazu bestimmt ist, die Depigmentierung der Haut zu begünstigen.

**3.** Verwendung gemäß einem der Ansprüche 1 und 2, bei der diese Verbindungen zur Behandlung brauner Flecken an der Hautoberfläche bestimmt sind.

**4.** Verwendung nach einem der Ansprüche 1 bis 3, bei der die Konzentration an sophorolipiden Verbindungen zwischen 0,01 bis 35 Gew.-% Trockenmasse beträgt.